# EUROPÄISCHE PATENTSCHRIFT

(11) **EP 1 562 660 B1**
(45) Veröffentlichungstag und Bekanntmachung des Hinweises auf die Patenterteilung: **31.05.2006**
(21) Anmeldenummer: 03763564.6
(22) Anmeldetag: 08.07.2003
(51) Int. Cl.: A61M 5/315

(54) **VERABREICHUNGSGERÄT MIT VERSTELLBAREM DOSIERANSCHLAG**
ADMINISTRATION DEVICE COMPRISING AN ADJUSTABLE DOSAGE STOP
APPAREIL D'ADMINISTRATION A BUTEE DE DOSAGE REGLABLE

(30) Priorität: 17.07.2002 DE 10232411
(43) Veröffentlichungstag der Anmeldung: 17.08.2005
(73) Patentinhaber: TecPharma Licensing AG, 3401 Burgdorf (CH)
(72) Erfinder: GRAF, Roney, CH-3400 Burgdorf (CH); KIRCHHOFER, Fritz, CH-3454 Sumiswald (CH)
(86) Internationale Anmeldenummer: PCT/CH2003/000458
(87) Internationale Veröffentlichungsnummer: WO 2004/007001

(56) Entgegenhaltungen:
- US-A- 5 304 152
- US-B1- 6 221 046
- US-B1- 6 228 067
- US-B1- 6 241 709

## Beschreibung

Die Erfindung betrifft ein Verabreichungsgerät, das zur Auswahl einer auszuschüttenden Dosis eines Produkts einen verstellbaren Dosieranschlag umfasst.

Die WO 97/36625 und die DE 199 00 792 C1 beschreiben Injektionsgeräte mit einem ein Reservoir aufnehmenden ersten Gehäuseabschnitt und einem zweiten Gehäuseabschnitt, und, die für eine Dosisauswahl je ein Dosierglied umfassen, das einen Dosieranschlag für eine Antriebsvorrichtung bildet. Die jeweilige um zweiten Gehäuseabschnitt befreidliche Antriebsvorrichtung wirkt auf eine Kolbenstange des Geräts, die ihrerseits auf einen Kolben in einem Produktreservoir wirkt, um für eine Ausschüttung der ausgewählten Produktdosis den Kolben im Reservoir auf einen Reservoirauslass zu vorzuschieben. Durch die Verstellung des Dosieranschlags wird der Hub der Antriebsvorrichtung und damit zusammen der Hub der Kolbenstange bestimmt. Die beiden bekannten Dosierglieder sind hülsenförmig und mit einem Gehäuse des jeweiligen Injektionsgeräts um ihre Längsachse drehbar verbunden, um durch eine Drehbewegung des Dosierglieds den Dosieranschlag und damit den Hub der Antriebsvorrichtung einzustellen. Während nach der WO 97/36625 der Dosieranschlag um die Längsachse des Dosierglieds treppenförmig umläuft, wird der Dosieranschlag des Dosierglieds nach der DE 199 00 792 Cl von einer spiraligen Anschlagfläche gebildet, die kontinuierlich um die Längsachse mit einer in Längsrichtung gesehen konstanten Steigung umläuft.

Das Injektionsgerät der WO 97/36625 erfordert bei der Antriebsvorrichtung die Ausbildung eines Anschlagnockens, der für das Zusammenwirken mit dem Dosieranschlag wegen dessen Stufigkeit und feinen Teilung in Umfangsrichtung recht schmal sein muss. Es besteht daher die Gefahr, dass der schmale Anschlagnocken durch wiederholten Anschlag gegen den Dosieranschlag abgeschert werden könnte. Der kontinuierlich schräge Verlauf des Dosieranschlags bei dem Injektionsgerät der DE 199 00 792 C1 erfordert Maßhaltigkeit nicht nur in Längsrichtung bzw. Vorschubrichtung der Antriebsvorrichtung, sondern wegen der kontinuierlichen Anschlagkurve in besonderem Maße auch in Bezug auf die Drehwinkelposition des Dosierglieds.

Aus der US 6,228,067 B1 ist ein Injektionsgerät bekannt, das einen vorderen Gehäuseabschnitt und einen hinteren Gehäuseabschnitt aufweist, die miteinander verdrehbar, aber verschiebegesichert verbunden sind. Der vordere Gehäuseabschnitt nimmt das Reservoir auf, und der hintere Gehäuseabschnitt lagert eine Betätigungseinrichtung, durch deren Betätigung eine zuvor ausgewählte Produktdosis ausschüttbar ist. Für die Auswahl der Produktdosis ist ein Dosierglied vorgesehen, das mit dem vorderen Gehäuseabschnitt über ein Schraubgelenk verbunden ist und von dem hinteren Gehäuseabschnitt geradgeführt wird. Durch eine Relativdrehung zwischen den beiden Gehäuseabschnitten wird das Dosierglied daher relativ zu dem hinteren Gehäuseabschnitt geradverschoben. Bei dieser Relativverschiebung wird das Dosierglied im gleichen Ausmaß auch relativ zu der Betätigungseinrichtung verschoben, wodurch ein mit der Betätigungseinrichtung ausführbarer Ausschütthub bestimmt wird. Die Länge des Ausschütthubs entspricht der ausgewählten Produktdosis. Die Betätigungseinrichtung weist elastisch biegbare Klauen auf, die in Zahnreihen einer als Zahnstange gebildeten Kolbenstange eingreifen, so dass die Betätigungseinrichtung bei ihrem Ausschütthub die Kolbenstange mitnimmt. Die elastischen Klauen müssen gegen ihre elastischen Rückstellkräfte in den Mitnahmeeingriff mit der Kolbenstange bewegt werden. Dies wird mittels des Dosierglieds bewirkt. Das Dosierglied bildet einen Dosieranschlag für die Betätigungseinrichtung, indem die Klauen in der Anschlagposition von der Kolbenstange abgerückt entspannt an dem Dosieranschlag des Dosierglieds anliegen. Führt die Betätigungseinrichtung ihren Ausschütthub aus, so werden die elastischen Klauen an dem Dosierglied entlang bewegt und dabei zwangsweise gegen ihre elastischen Rückstellkräfte in den die Mitnahme bewirkenden Eingriff mit der Kolbenstange gedrückt. Die Dosiermechanik ist komplex, da sie mit den beiden Gehäuseabschnitten und dem Dosierglied drei zueinander bewegbare Körper erforderlich macht, deren Bewegungen relativ zueinander zum einen über ein Schraubgelenk und zum anderen über die Geradführung koordiniert werden müssen.

Es ist eine Aufgabe der Erfindung, für Komponenten eines Verabreichungsgeräts, die an einer Dosisauswahl beteiligt sind, die mechanische Stabilität und die Maßhaltigkeit zu verbessern.

Die Erfindung betrifft ein Verabreichungsgerät zur Verabreichung eines fluiden Produkts. Das Verabreichungsgerät umfasst ein Gehäuse, ein Reservoir für das Produkt, eine Fördereinrichtung, und ein Dosierglied für die Auswahl einer Produktdosis, die im Zuge einer Injektion aus dem Reservoir ausgeschüttet wird. Das Reservoir wird vorzugsweise von einem Behältnis, beispielsweise einer Ampulle, gebildet, die von einem ersten Abschnitt des Gehäuses aufgenommen wird. Grundsätzlich kann jedoch auch der erste Gehäuseabschnitt das Reservoir unmittelbar bilden.

Die Fördereinrichtung umfasst eine Antriebsvorrichtung und eine Abtriebsvorrichtung. Die Abtriebsvorrichtung wirkt auf das in dem Reservoir enthaltene Produkt, um es durch einen Auslass des Reservoirs auszuschütten. Die Antriebsvorrichtung ist mit der Abtriebsvorrichtung gekoppelt, um sie für die Produktausschüttung anzutreiben. Die Antriebsvorrichtung wird von einem zweiten Gehäuseabschnitt aufgenommen, der mit dem ersten Gehäuseabschnitt vorzugsweise nicht bewegbar verbunden ist und sogar in einem Stück mit dem ersten Gehäuseabschnitt gebildet sein kann.

In besonders bevorzugter Ausführung wird das Produkt mit Hilfe eines Kolbens gefördert. Die Abtriebsvorrichtung umfasst in dieser Ausführung demgemäß den Kolben und eine auf den Kolben wirkende Kolbenstange. Der Kolben ist in dem Reservoir in eine Vorschubrichtung auf einen Auslass des Reservoirs zu bewegbar, um die ausgewählte Produktdosis durch den Auslass hindurch auszuschütten. Bewegungen in und gegen die Vorschubrichtung des Kolbens werden im Folgenden auch als axiale Bewegungen bezeichnet. Der Kolben und die Kolbenstange können aus einem Stück oder separat gefertigt und anschließend für die Erfüllung ihrer Funktion ausreichend fest miteinander verbunden sein. Vorzugsweise werden sie jedoch von separaten Teilen gebildet, so dass die Kolbenstange in Vorschubrichtung gegen den Kolben drückt, um ihn für die Produktausschüttung vorzuschieben.

Das Dosierglied bildet einen Dosieranschlag für die Antriebsvorrichtung. Es ist mit dem Gehäuse bewegbar gekoppelt, um durch eine Verstellung des Dosieranschlags die Auswahl der auszuschüttenden Produktdosis vornehmen zu können.

Die Antriebsvorrichtung ist relativ zu dem Gehäuse in eine Antriebsrichtung bis gegen einen Ausschüttanschlag und gegen die Antriebsrichtung bis gegen den Dosieranschlag bewegbar. Der Ausschüttanschlag ist relativ zu dem Reservoirauslass in und gegen die Antriebsrichtung fix, zumindest während der Bewegung der Antriebsvorrichtung in die Antriebsrichtung, vorzugsweise ist er in und gegen die Antriebsrichtung relativ zu dem Gehäuse stets unbeweglich. Er wird vorzugsweise von dem Gehäuse unmittelbar gebildet. Durch die Verstellung des Dosieranschlags wird ein Ausschütthub der Antriebsvorrichtung vorgegeben.

Die Antriebsvorrichtung und die Kolbenstange, falls ein Kolben für die Förderung verwendet wird, sind so miteinander gekoppelt, dass die Antriebsvorrichtung bei ihrem Ausschütthub, d.h. bei ihrer Bewegung in die Antriebsrichtung, die Kolbenstange in die Vorschubrichtung bewegt. Andererseits wird jedoch die Kolbenstange an einer Bewegung relativ zu dem Gehäuse gegen die Vorschubrichtung gehindert. Hierfür weist das Verabreichungsgerät eine Sperreinrichtung auf, die von dem Gehäuse unmittelbar gebildet oder von dem Gehäuse axial fix gelagert wird. Die Antriebsvorrichtung führt somit den Ausschütthub von dem Dosieranschlag bis gegen den Ausschüttanschlag zusammen mit der Kolbenstange und einen Aufziehhub oder "Ladehub" in die Gegenrichtung von dem Ausschüttanschlag bis gegen den Dosieranschlag ohne die Kolbenstange aus. Vorzugsweise stimmen die Antriebsrichtung und die Vorschubrichtung überein und die Bewegung der Antriebsvorrichtung und der Kolbenstange verlaufen während des Ausschütthubs entlang einer gemeinsamen Achse, d.h. die Antriebsvorrichtung nimmt bei ihrem Ausschütthub die Kolbenstange mit.

Nach der Erfindung ist ein Dosierglied für die Verstellung des Dosieranschlags in die Antriebsrichtung und/oder gegen die Antriebsrichtung relativ zu dem zweiten Gehäuseabschnitt translatorisch entlang einer Translationsachse und rotatorisch bewegbar, wobei die Rotation und die Translation gekoppelt sind. Es wird, mit anderen Worten, das Dosierglied mit dem von ihm gebildeten Dosieranschlag im Ganzen rotatorisch und gleichzeitig in fest vorgegebener Weise translatorisch bewegt.

Durch die translatorische Bewegung des Dosierglieds kann der Dosieranschlag vorteilhafterweise eine in Bezug auf die Translationsachse konstante Höhe um die Translationsachse einnehmen. Ein von der Antriebsvorrichtung gebildeter Gegenanschlag für den Dosieranschlag wird daher nicht wie im Falle eines treppenförmigen Dosieranschlags durch die Teilung der Treppenstufen in Bezug auf seine Erstreckung in Umfangsrichtung begrenzt. Um diesen Vorteil zu erhalten, muss der Dosieranschlag nicht unbedingt als vollständig um die Translationsachse umlaufende Anschlagfläche gebildet sein. Er kann durchaus unterbrochen und dadurch kronenförmig sein. Er muss sich auch nicht unbedingt über einen vollen Umlauf von 360° erstrecken, sondern nur über einen Teil eines vollen Umlaufs. Allerdings ist darauf zu achten, dass der Dosieranschlag in Umfangsrichtung nicht allzu schmal ist, weil er dann seinerseits durch den anschlagenden Gegenanschlag beschädigt, insbesondere abgebrochen werden könnte. Wegen der translatorischen Verstellung des Dosieranschlags muss für die Maßhaltigkeit in Bezug auf die Länge zwischen dem Ausschüttanschlag und dem Dosieranschlag beim Dosierglied auch keine Drehwinkelposition berücksichtigt werden. Hierdurch wird zusätzlich auch die Toleranzanalyse gegenüber einer kontinuierlich umlaufenden schrägen Anschlagkurve vereinfacht und die Maßhaltigkeit der an der Dosierung beteiligten Komponenten verbessert. Der erfindungsgemäß rotationsinvariante Dosieranschlag erlaubt im Vergleich zum treppenförmigen Dosieranschlag daher eine mechanisch stabilere Konstruktion des Gegenanschlags der Antriebsvorrichtung und schafft eine Erleichterung für die Qualitätskontrolle und eine Verbesserung der Maßhaltigkeit im Vergleich zur kontinuierlichen, schrägen Anschlagkurve.

Für die Ausführung der kombinierten Rotations- und Translationsbewegung ist das Dosierglied mit dem zweiten Gehäuseabschnitt mittels vorzugsweise eines einzigen Gelenks oder gegebenenfalls mehrerer Gelenke so gekoppelt, dass die Rotationsbewegung des Dosierglieds seine Translationsbewegung bewirkt. Die Kopplung des Dosierglieds mit dem Gehäuse kann über ein Übertragungsglied oder mehrere Übertragungsglieder erfolgen. Bevorzugter sind das Dosierglied und das Gehäuse jedoch unmittelbar miteinander in einem Eingriff und bilden selbst Gelenkelemente, bevorzugt die einzigen Gelenkelemente, des Gelenks.

Obgleich nicht unumgänglich erforderlich, wird es jedoch bevorzugt, dass die Rotationsachse der Rotationsbewegung des Dosierglieds und die Translationsachse des Dosierglieds identisch sind. Besonders bevorzugt sind das Dosierglied und das Gehäuse miteinander in einem Gewindeeingriff, um die Rotationsbewegung des Dosierglieds in die Translationsbewegung umzuwandeln. Der Gewindeeingriff kann von einem einzigen, vollständig umlaufenden Gewindezug oder auch nur von einem teilweise umlaufenden Gewindezug gebildet werden. Für die Feinheit der Dosierung ist es jedoch vorteilhafter, wenn die im Gewindeeingriff befindlichen Gelenkelemente des Schraubgelenks, besonders bevorzugt das Dosierglied und das Gehäuse unmittelbar, mit einer Mehrzahl von vollständig umlaufenden Gewindezügen in dem Gewindeeingriff sind.

In der besonders bevorzugten Ausbildung des Gelenks als Schraubgelenk und Ausbildung des Dosierglieds als Gelenkelement kann ein derartiges Dosierglied auch als Dosierschraube bezeichnet werden.

Da das Dosierglied relativ zu dem hinteren Gehäuseabschnitt eine kombinierte Rotations- und Translationsbewegung ausführt, kann eine Dosisskala, auf der die ausgewählte Produktdosis ablesbar ist, wie bei dem Injektionsgerät der US 6,228,067 B1 sowohl in die Richtung der relativen Rotation als auch in die Richtung der relativen Translation des Dosierglieds erstreckt werden. Andererseits müssen ganze Gehäuseabschnitte, die jeweils Teile des Verabreichungsgeräts aufnehmen, zum Zwecke der Dosisauswahl nicht relativ zueinander bewegt werden. Die Dosisanzeige kann darüber hinaus vereinfacht werden, da die ausgewählte Dosis bereits allein aus der Position abgelesen werden kann, die das Dosierglied relativ zu dem hinteren Gehäuseabschnitt einnimmt, wodurch jedoch nicht ausgeschlossen sein soll, dass an der Dosisanzeige auch noch andere Komponenten des Verabreichungsgeräts beteiligt sein können. Es muss auch nicht unumgänglich der hintere Gehäuseabschnitt zumindest bereichsweise durchsichtig sein, wie bei dem Injektionsgerät der US 6.228,067 B1, obgleich dies auch nach der Erfindung zur Erzielung von besonderen Vorteilen der Fall sein kann.

In bevorzugter Ausführung umfasst das Dosierglied einen inneren Dosierkörper und einen äußeren Dosierkörper, zwischen denen in einem Überlappungsbereich ein lichter Spalt verbleibt. Der innere Dosierkörper ragt in das Gehäuse hinein. Das Gehäuse ragt in den lichten Spalt hinein. Der äußere Dosierkörper kann insbesondere ein Griffteil des Dosierglieds bilden. Die Anordnung der Dosierkörper ist so, dass das Dosierglied um die Translationsachse relativ zu dem in den Spalt eingreifenden Gehäuse verdreht werden kann. Die geschachtelte Anordnung eines inneren Dosierkörpers und eines äußeren Dosierkörpers erlaubt eine axial kurze Bauweise des Dosierglieds. Durch eine bevorzugte separate Fertigung des inneren Dosierkörpers und des äußeren Dosierkörpers kann das Dosierglied einfacher als im Falle einer einstückigen Fertigung mit einer höheren Funktionalität ausgestattet werden. Im Falle der separaten Fertigung sind der innere Dosierkörper und der äußere Dosierkörper vorzugsweise so miteinander verbunden, dass zwischen den Dosierkörpern keine Translationsbewegung entlang der Translationsachse und keine Rotationsbewegung um die Translationsachse möglich ist, sondern der eine der Dosierkörper den anderen mitnimmt.

Als besonderen Vorteil ermöglicht die Erfindung in einer Weiterbildung ein einfaches Primen des Verabreichungsgeräts. Als Primen wird ein Vorgang bezeichnet, durch den das Reservoir und, soweit vorhanden, die fluidführenden Teile des Verabreichungsgeräts, die sich an den Auslass des Reservoirs in Strömungsrichtung anschließen, durch Förderung des Produkts entlüftet wird bzw. werden.

Für das Primen ist es vorteilhaft, dass das Dosierglied und die Antriebsvorrichtung in jeder Dosierposition des Dosierglieds relativ zueinander in Bezug auf die Antriebsrichtung die gleiche Position einnehmen, wenn die Antriebsvorrichtung und der Dosieranschlag auf Anschlag sind. In diesem Fall kann nämlich die Antriebsvorrichtung zum Zwecke des Primens mit dem Dosierglied so gekoppelt werden, dass die Länge des Hubs, den die Antriebsvorrichtung für das Primen durchführt, mittels der Kopplung aus der Anschlagposition exakt vorgegeben wird.

Nach der Erfindung sind ein Priminganschlag und ein Priminggegenanschlag vorgesehen, von denen der eine mit der Antriebsvorrichtung und der andere mit dem Gehäuse oder bevorzugter mit dem Dosierglied verbunden ist. Der Priminggegenanschlag ist relativ zu dem Priminganschlag hin und her bewegbar zwischen einer Ausschüttposition und einer Primingposition. Aus der Ausschüttposition kann die Ausschüttbewegung der Antriebsvorrichtung vorgenommen werden. In der Primingposition liegen sich der Priminganschlag und der Priminggegenanschlag in Antriebsrichtung in einem Abstand gegenüber, der kürzer ist als der Hub, den die Antriebsvorrichtung aus der Ausschüttposition ausführen kann.

Die Auswahlbewegung des Priminggegenanschlags von der Ausschüttposition in die Primingposition und eine Rückführbewegung in die Ausschüttposition sind vorzugsweise je eine Rotationsbewegung, die der Priminggegenanschlag relativ zu dem Priminganschlag um die Translationsachse des Dosierglieds ausführt. Diese Auswahlbewegung zwischen Ausschütten und Primen kann zweckmäßigerweise ausgeführt werden, wenn die Antriebsvorrichtung sich in ihrer vorderen Anschlagposition gegen den Ausschüttanschlag oder in ihrer hinteren Anschlagposition gegen den Dosieranschlag befindet. Falls die erstere Ausbildung zum Einsatz kommt, wird der Priminganschlag vorzugsweise von dem Gehäuse gebildet. In der besonders bevorzugten Ausbildung, in der die Auswahlbewegung in der hinteren Anschlagposition der Antriebsvorrichtung stattfindet, wird der Priminganschlag vorzugsweise von dem Dosierglied gebildet.

In bevorzugter Ausführung werden der Priminganschlag oder der Priminggegenanschlag von einer Führungskurve und der andere der beiden Anschläge von einem in die Führungskurve eingreifenden Eingriffsglied gebildet. Die Führungskurve und das Eingriffsglied bilden eine Führungskulisse. Die Führungskurve und das Eingriffsglied sind vorzugsweise an einander zugewandten Mantelflächen von zwei Körpern einer Primingmechanik geformt. Vorzugsweise bilden das Dosierglied und die Antriebsvorrichtung diese beiden Körper. Einer bevorzugten Ausführung entspricht es ferner auch, wenn das Gehäuse und die Antriebsvorrichtung diese beiden Körper bilden. Die Führungskurve weist zwei unterschiedlich lange, in Antriebsrichtung weisende Führungsabschnitte auf, die vorzugsweise parallel voneinander beabstandet an einer Innenmantelfläche oder Außenmantelfläche des einen der unmittelbar in dem Eingriff befindlichen Körper gebildet sind. Eine Stirnseite des kürzeren der beiden Axialabschnitte bildet den Priminganschlag. Die beiden Führungsabschnitte werden von einem Verbindungsabschnitt der Führungskurve miteinander verbunden. Das Eingriffsglied kann innerhalb des Verbindungsabschnitts die Auswahlbewegung ausführen, d.h. wahlweise entweder in den längeren oder den kürzeren der Führungsabschnitte bewegt werden. Zur Durchführung der Ausschüttbewegung wird das Eingriffsglied bis in die Ausschüttposition bewegt, in der es in Antriebsrichtung gesehen in der Flucht des längeren der Führungsabschnitte zu liegen kommt.

Für das Primen wird das Eingriffsglied in die Primingposition bewegt, in der es in Antriebsrichtung gesehen in der Flucht des kürzeren der beiden Führungsabschnitte und damit in die Flucht zu dem Priminganschlag zu liegen kommt. Für die Auswahlbewegung ist wesentlich, dass sie zwischen der Führungskurve und dem Eingriffsglied stattfindet. Relativ zu dem Gehäuse kann zu ihrer Ausführung die Führungskurve oder das Eingriffsglied bewegt werden; es können auch beide relativ zueinander und relativ zu dem Gehäuse bewegt werden, obgleich dies weniger bevorzugt ist.

In bevorzugter Ausführung ist das Dosierglied Bestandteil einer Dosiereinrichtung, die für eine optische Anzeige der ausgewählten Produktdosis auch eine Dosisskala umfasst. Die Dosisskala kann mit dem Dosierglied verbunden sein. Bevorzugter ist sie jedoch mit dem Gehäuse verbunden. Insbesondere kann das Gehäuse unmittelbar Träger der Dosisskala sein. Das Dosierglied bildet in diesem Fall einen Zeiger der optischen Anzeige, dessen Position relativ zu der Dosisskala die ausgewählte Produktdosis anzeigt. Der Zeiger des Dosierglieds wird vorzugsweise von einem Sichtfenster gebildet. Das Sichtfenster ist vorzugsweise zu einer Lupe weitergebildet. Die durch das Sichtfenster ablesbare Dosiseinheit der Dosisskala gibt die ausgewählte Produktdosis an.

In einer besonders bevorzugten Weiterbildung ist das Gehäuse wenigstens in einem Gehäusebereich, der die Dosisskala oder eine weitere Dosisskala überdeckt, durchsichtig. Die Dosisskala kann an einer Oberfläche des durchsichtigen Gehäusebereichs angebracht, beispielsweise aufgedruckt sein. Sie kann in dem durchsichtigen Gehäusebereich auch in dem Gehäusematerial gebildet sein, beispielsweise mittels Laserbeschriftung eines hierfür geeigneten, durchsichtigen Gehäusematerials. Grundsätzlich kann das Gehäuse zum Erhalt der Durchsichtigkeit auch mit einem Durchbruch versehen sein, durch den die Dosisskala abgelesen werden kann. Die zumindest bereichsweise Durchsichtigkeit des Gehäuses gestattet die optische Anzeige der tatsächlichen Position desjenigen Teils der Antriebsvorrichtung, der in dem Eingriff mit der Kolbenstange ist. Für diese Anzeige bildet die Antriebsvorrichtung einen Zeiger, dessen auf die Antriebsrichtung bezogene Position zwischen dem Ausschüttanschlag und dem Dosieranschlag in dem durchsichtigen Gehäusebereich auf der Dosisskala abgelesen werden kann.

Obgleich die beiden genannten Skalen, nämlich die Dosisskala für die Anzeige der Position der Antriebsvorrichtung und die Dosisskala zur Anzeige der ausgewählten Produktdosis, je von einer separaten Dosisskala gebildet werden können, werden sie bevorzugterweise von ein und derselben Dosisskala gebildet. Bildet in diesem Fall auch noch eine Lupe den genannten Zeiger des Dosierglieds, so kann der Verwender besonders einfach und genau verifizieren, ob die Antriebsvorrichtung auch tatsächlich diejenige Position einnimmt, die der ausgewählten und mit Hilfe der Lupe angezeigten Produktdosis entspricht.

In bevorzugten Ausführungen wird die Kolbenstange von dem Gehäuse so gelagert, dass sie zwar in die Vorschubrichtung, aber nicht gegen die Vorschubrichtung bewegt werden kann. Eine Bewegung gegen die Vorschubrichtung ist in diesem Fall allenfalls im Zuge eines Reservoirwechsels durch entsprechende Montagehandgriffe möglich. In einfachen und besonders bevorzugten Ausführungen ist eine Rückführung der Kolbenstange jedoch nicht möglich. Das Verabreichungsgerät wird vielmehr gänzlich durch ein neues ersetzt. Denkbar wäre auch die Aufspaltung des Geräts in ein als Verbrauchsmodul konzipiertes Reservoirmodul und ein Dosier- und Antriebsmodul, das wiederholt mit neuen Reservoirmodulen verwendbar ist. Dieses Konzept wird bei Injektionsgeräten bereits mit Erfolg in Form von sogenannten semidisposable Injektionspens verfolgt und ist auch für das Verabreichungsgerät der Erfindung mit Vorteil einsetzbar.

Das fluide Produkt ist bevorzugt eine Flüssigkeit für medizinische, therapeutische, diagnostische, pharmazeutische oder kosmetische Anwendungen oder kombiniert für mehrere dieser Anwendungen. Das Produkt kann beispielsweise Insulin, ein Wachstumshormon, Cortison, Collagen oder auch eine Flüssignahrung sein. Das Verabreichungsgerät wird vorzugsweise in solchen Verwendungen eingesetzt, in denen ein Verwender sich das Produkt selbst verabreicht, wie es beispielsweise in der Diabetestherapie üblich ist. Eine Verwendung nur oder auch im stationären und ambulanten Bereich durch geschultes Personal soll jedoch nicht ausgeschlossen sein.

Die Produktverabreichung kann im Falle eines Injektionsgeräts mittels Injektionskanüle oder beispielsweise einer Düse für nadellose Injektionen erfolgen. Die Injektion kann insbesondere subkutan oder venös vorgenommen werden, oder auch intramuskulär. Das Verabreichungsgerät kann auch ein Inhalationsgerät sein, bei dem die ausgewählte Produktdosis oder eine im Reservoir noch vorhandene Restdosis aus dem Reservoir beispielsweise in eine Kammer des Inhalationsgeräts ausgeschüttet und mittels einer Zerstäubungseinrichtung oder einer sonstigen Vemebelungseinrichtung für die Inhalation vernebelt wird. Denkbar ist ferner eine orale Einnahme des Produkts oder eine Verabreichung über die Speiseröhre, um nur einige Beispiele der Verabreichung zu nennen.

In den Unteransprüchen werden weitere bevorzugte Ausgestaltungen der Erfindung beschrieben.

Ein Ausführungsbeispiel der Erfindung wird nachfolgend anhand von Figuren beschrieben. Die an dem Ausführungsbeispiel offenbar werdenden Merkmale bilden je einzeln und in jeder Merkmalskombination die Gegenstände der Ansprüche weiter. Es zeigen:
- Figur 1: ein Injektionsgerät in einem Längsschnitt,
- Figur 2: den hinteren Teil des Injektionsgeräts,
- Figur 3: das Injektionsgerät in einem Querschnitt,
- Figur 4: ein Dosierglied des Injektionsgeräts in einem Längsschnitt,
- Figur 5: ein modifiziertes Dosierglied in einem Längsschnitt,
- Figur 6: den hinteren Teil des Injektionsgeräts in einem Minimaldosis-Zustand und "nicht geladen",
- Figur 7: den hinteren Teil des Injektionsgeräts in einem Maximaldosis-Zustand und "nicht geladen",
- Figur 8: den hinteren Teil des Injektionsgeräts in dem Maximaldosis-Zustand und "geladen",
- Figur 9: eine Ansicht auf eine Dosisanzeige in dem Gerätezustand der Figur 6,
- Figur 10: die Ansicht auf die Dosisanzeige nach einem "Ladevorgang" aus dem Zustand der Figur 9,
- Figur 11: die Ansicht auf die Dosisanzeige in dem Gerätezustand der Figur 7,
- Figur 12: die Ansicht auf die Dosisanzeige in dem Gerätezustand der Figur 8 und
- Figur 13: eine Dosisskala abgewickelt in die Blattebene.

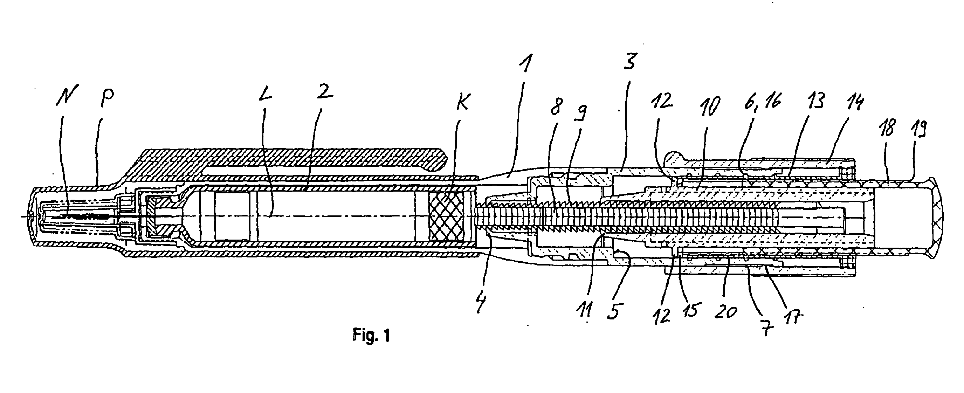

Figur 1 zeigt in einem Längsschnitt ein Injektionsgerät komplett. Es handelt sich um einen Zahnstangenpen für die Selbstverabreichung von beispielsweise Insulin oder Wachstumshormonen. Ein erster vorderer Gehäuseabschnitt 1 und ein zweiter, hinterer Gehäuseabschnitt 3 bilden eine Gehäuse des Pens. Die Gehäuseabschnitte 1 und 3 sind Hülsenkörper. Zur Bildung des Gehäuses sind sie entlang einer gemeinsamen Mittellängsachse L ineinander gesteckt und unlösbar verbunden. Die Verbindung ist ferner so, dass die Gehäuseabschnitte 1 und 3 relativ zueinander weder axial noch rotativ um die Längsachse L bewegt werden können.

Eine Ampulle 2, die in dem vorderen Gehäuseabschnitt 1 aufgenommen ist, bildet ein Reservoir für ein Produkt, das mit dem Pen im Wege einer Injektion verabreicht wird. Ein Auslass der Ampulle 2 wird von einer Membran verschlossen. Allerdings ist eine Injektionsnadel N durch die Membran geführt und ragt mit einem hinteren Ende in die Ampulle 2 hinein. An einem von dem Auslass abgewandten, hinteren Ende der Ampulle 2 ist ein Kolben K aufgenommen. Durch eine axiale Bewegung des Kolbens K entlang der Längsachse L in eine auf den Ampullenauslass weisende Vorschubrichtung wird Produkt aus der Ampulle 2 und durch die Injektionsnadel N hindurch ausgeschüttet und dadurch verabreicht. Im dargestellten Zustand sind der vordere Gehäuseabschnitt 1 von einer äußeren Schutzkappe P und die Injektionsnadel N noch einmal separat von einer inneren Schutzkappe abgedeckt, die vor der Verabreichung selbstverständlich abgenommen werden müssen.

Der hintere Gehäuseabschnitt 3 bildet einen Mechanikhalter, indem er eine auf den Kolben K in Vorschubrichtung wirkende Kolbenstange 8 und die weiteren Komponenten des Pens lagert, die an einer Auswahl einer auszuschüttenden Produktdosis und einer Ausschüttung der ausgewählten Produktdosis beteiligt sind. Diese weiteren Komponenten sind ein Antriebsglied 10 und ein Betätigungselement 18, die zusammen eine Antriebsvorrichtung 10/18 für die Kolbenstange 8 bilden, und ferner ein Dosierglied 13/14, das im Zusammenwirken mit dem hinteren Gehäuseabschnitt 3 der Auswahl der aus der Ampulle 2 auszuschüttenden Produktdosis dient.

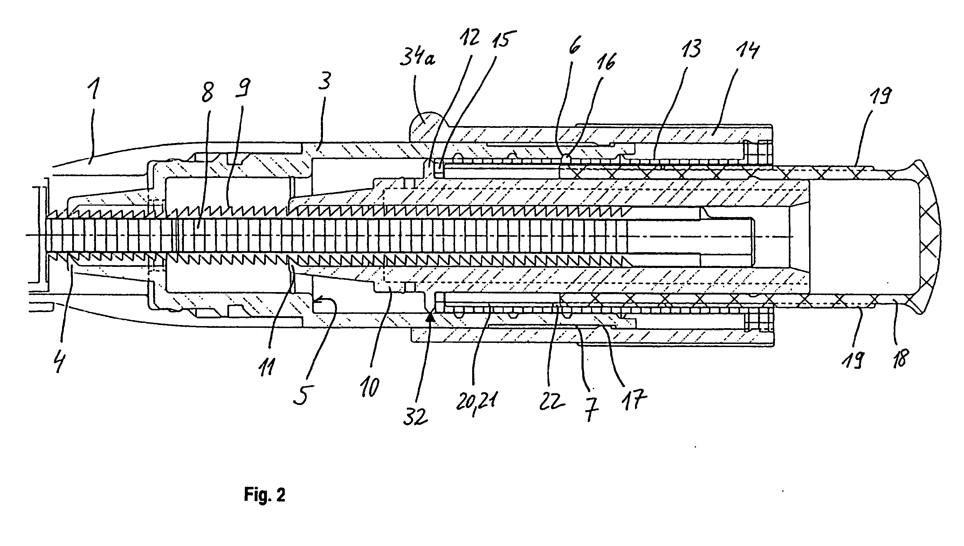

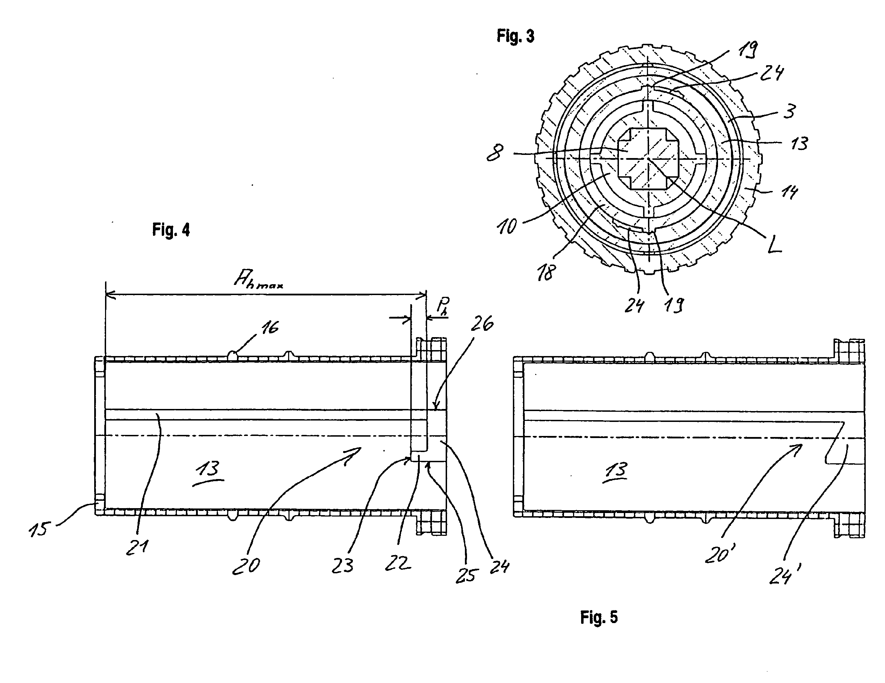

In Figur 2 sind diese Komponenten und ihre Kopplungen vergrößert dargestellt. Auch auf die Querschnittsdarstellung der Figur 3 sei stets ergänzend hingewiesen.

An dem Außenmantel der Kolbenstange 8 erstrecken sich in Längsrichtung jeweils um 90° zueinander versetzt vier aus Sägezähnen gebildete Zahnreihen 9. Die Zahnreihen 9 weisen je die gleiche Teilung auf. Jede der Zahnreihen 9 ist gegenüber jeder der anderen Zahnreihen 9 um eine viertel Zahnteilung versetzt, um die Feinheit der Dosierung in an sich bekannter Weise zu verbessern.

Der hintere Gehäuseabschnitt 3 bildet Sperrelemente 4, die in die Zahnreihen 9 eingreifen. Durch den Eingriff der Sperrelemente 4 wird die Kolbenstange 8 so blockiert, dass eine Bewegung der Kolbenstange 8 gegen die Vorschubrichtung des Kolbens K nicht möglich ist. Die Bewegung der Kolbenstange 8 in die Vorschubrichtung wird allerdings zugelassen.

Um den Vorschub der Kolbenstange 8 zu bewirken, greift hinter den Sperrelementen 4 die Antriebsvorrichtung 10/18 mit Mitnehmern 11 in die Zahnreihen 9 der Kolbenstange 8 ein. Die Antriebsvorrichtung 10/18 wird von dem hülsenförmigen Antriebsglied 10 und dem hülsenförmigen Betätigungselement 18 gebildet. Das Betätigungselement 18 ist von hinten auf das Antriebsglied 10 aufgeschoben. Die Verbindung zwischen dem Antriebsglied 10 und dem Betätigungselement 18 ist derart, dass das Betätigungselement 18 relativ zu dem Antriebsglied 10 axial nicht bewegbar, aber um die Längsachse L relativ zu dem Antriebsglied 10 drehbar ist. Die Längsachse L bildet eine Translationsachse der Antriebsvorrichtung 10/18 und der aus dem Kolben K und der Kolbenstange 8 bestehenden Abtriebsvorrichtung K, 8 und ferner eine Rotationsachse des Betätigungselements 18.

Die Antriebsvorrichtung 10/18 im Ganzen gesehen durchragt das Dosierglied 13/14. Die Mitnehmer 11 des Antriebsglieds 10 können frei, insbesondere frei von dem Dosierglied 13/14, in radialer Richtung elastisch nach außen aus dem Zahneingriff abgebogen werden, um eine Bewegung der Antriebsvorrichtung 10/18 relativ zu der Kolbenstange 8 entgegen der Vorschubrichtung zu ermöglichen. Die Mitnehmer 11 sind in einem nach vorn aus dem Dosierglied 13/14 ragenden Abschnitt der Antriebsvorrichtung 10/18 gebildet; im Ausführungsbeispiel bilden sie das vordere Ende der Antriebsvorrichtung 10/18. Der Eingriff der Mitnehmer 11 und die Form der Zahnreihen 9 sind derart, dass die Kolbenstange 8 durch eine Bewegung der Antriebsvorrichtung 10/18 in eine auf den Kolben K gerichtete Antriebsrichtung, die mit der Vorschubrichtung identisch ist, zwangsweise mitgenommen wird.

Im Ausführungsbeispiel sind die Zahnreihen 9 sägezahnförmig in Vorschubrichtung gepfeilt. In modifizierten Ausbildungen könnte die Kolbenstange 8 jedoch statt einer oder mehreren Zahnreihen 9 Vertiefungen oder andere Eingriffseinrichtungen für Mitnehmer einer Antriebsvorrichtung aufweisen, wenn nur sichergestellt ist, dass eine zwangsweise Mitnahme in die Vorschubrichtung erfolgt, aber eine Mitnahme gegen die Vorschubrichtung verhindert wird.

Das Dosierglied 13/14 wird von einem hülsenförmigen inneren Dosierkörper 13 und einem hülsenförmigen äußeren Dosierkörper 14 gebildet, die als separate Teile gefertigt und unbeweglich miteinander verbunden sind. Insbesondere ist zwischen den beiden Dosierkörpern 13 und 14 weder eine axiale Relativbewegung noch eine Relativdrehung um die Längsachse L möglich. Der äußere Dosierkörper 14 umgibt den inneren Dosierkörper 13 konzentrisch. Zwischen den beiden Dosierkörpern 13 und 14 verbleibt umlaufend ein lichter Ringspalt, in den der ebenfalls hülsenförmige hintere Gehäuseabschnitt 3 hineinragt.

Der hintere Gehäuseabschnitt 3 bildet mit dem Dosierkörper 13/14 ein Drehgelenk, genauer gesagt ein Schraubgelenk, welches bewirkt, dass eine Rotationsbewegung des Dosierglieds 13/14 um die Längsachse L in Abhängigkeit von der Drehrichtung eine Translationsbewegung des Dosierglieds 13/14 in oder gegen die Vorschubrichtung, also eine axiale Translationsbewegung, relativ zu dem hinteren Gehäuseabschnitt 3 zur Folge hat. Die Längsachse L bildet eine Rotationsachse und eine Translationsache des Dosierglieds 13/14. Zur Bildung des Drehgelenks sind der hintere Gehäuseabschnitt 3 und der Dosierkörper 13/14 in einem Gewindeeingriff. Der Gewindeeingriff besteht zwischen einem Innengewinde 6, das an einer Innenmantelfläche des hinteren Gehäuseabschnitts 3 geformt ist, und einem Außengewinde 16, das an einer Außenmantelfläche des inneren Dosierkörpers 13 geformt ist. Der Gewindeeingriff könnte, obgleich weniger bevorzugt, stattdessen auch zwischen einem Außengewinde des hinteren Gehäuseabschnitts 3 und einem eingreifenden Innengewinde des äußeren Dosierkörpers 13 gebildet sein. Durch Rotation des Dosierkörpers 13/14 wählt der Verwender die zu verabreichende Produktdosis. Indem durch den Gewindeeingriff das Dosierglied 13/14 gleichzeitig auch eine axiale Translationsbewegung ausführt, wird ein Ausschütthub der Länge Ah für die Antriebsvorrichtung 10/18 eingestellt, welcher der ausgewählten Produktdosis entspricht.

Der innere Dosierkörper 13 bildet einen Dosieranschlag 15, der die Bewegung der Antriebsvorrichtung 10/18 gegen die Vorschubrichtung begrenzt. Den Dosieranschlag 15 bildet genauer gesagt an seinem vorderen Ende eine in Vorschubrichtung weisende Stirnfläche des Dosieranschlags 15. Der Dosieranschlag 15 wird von einer um die Längsachse L umlaufenden Ringschulter gebildet, die nach radial einwärts über eine Innenmantelfläche des inneren Dosierkörpers 13 auf das Antriebsglied 10 zu vorsteht. Der Dosieranschlag 15, genauer gesagt dessen vordere Anschlagfläche, läuft auf einer axial konstanten Höhe um die Längsachse L um, d.h. es wird ein gerader Dosieranschlag 15 auf einer einzigen Höhe gebildet.

Dem Dosieranschlag 15 in Vorschubrichtung gegenüber bildet der hintere Gehäuseabschnitt 3 einen Ausschüttanschlag 5, der die Bewegung der Antriebsvorrichtung 10/18 in die Vorschubrichtung begrenzt. Die Antriebsvorrichtung 10/18 kann somit zwischen dem gehäusefesten Ausschüttanschlag 5, der sogar von dem hinteren Gehäuseabschnitt 3 selbst gebildet wird, und dem translativ verstellbaren Dosieranschlag 15 in und gegen die Vorschubrichtung bewegt werden. Die Antriebsvorrichtung 10/18 bildet ihrerseits an einer Außenmantelfläche des Antriebsglieds 10 einen Gegenanschlag, nämlich den Ausschütt- und Dosieranschlag 12. Der Ausschütt- und Dosieranschlag 12 wird von einer Ringschulter mit einer dem Ausschüttanschlag 5 zugewandten vorderen Anschlagfläche und einer dem Dosieranschlag 15 zugewandten hinteren Anschlagfläche gebildet. Der Ausschütt- und Dosieranschlag 12 steht von einer Außenmantelfläche des Antriebsglieds 10 radial nach außen auf eine zugewandte Innenmantelfläche des hinteren Gehäuseabschnitts 3 zu vor.

Der hintere Gehäuseabschnitt 3 ist gänzlich oder zumindest in dem Abschnitt durchsichtig, der einen maximalen Ausschütthub der Antriebsvorrichtung 10/18 überdeckt. Die Durchsichtigkeit dient der Verifizierung der axialen Position der Antriebsvorrichtung 10/18, insbesondere der Position des mit der Kolbenstange 8 unmittelbar in Eingriff stehenden und über den Eingriff in Vorschubrichtung steif verbundenen Antriebsglieds 10.

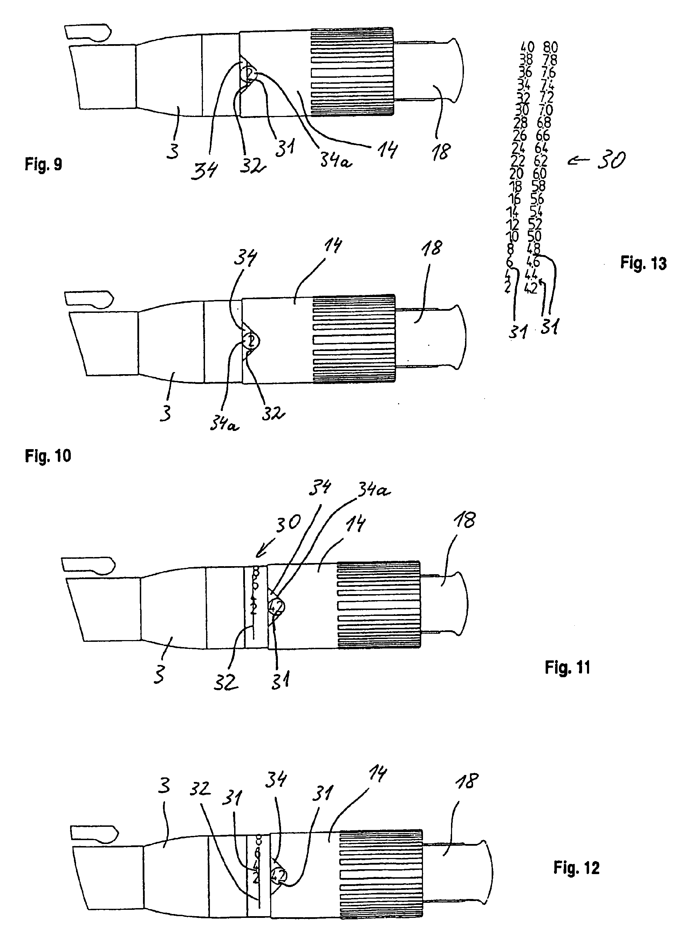

Zum Zwecke einer besonders genauen optischen Verifikation ist das Antriebsglied 10 mit einer dünnen Markierungslinie versehen, bevorzugt eine umlaufende Markierungslinie, die durch den hinteren Gehäuseabschnitt 3 hindurch gut sichtbar ist und einen Zeiger 32 bildet (Figuren 9 bis 11). Die Markierungslinie kann vorteilhafterweise von dem Ausschütt- und Dosieranschlag 12 selbst gebildet werden, indem der Ausschütt- und Dosieranschlag 12 radial außen eine dünne Spitze bildet. Der Ausschütt- und Dosieranschlag 12 bietet sich als Zeiger für die Verifikation bereits deshalb an, weil er radial bis fast unmittelbar an den hinteren Gehäuseabschnitt 3 ragt oder diesen sogar berührt und Gleitkontakt hat. Eine radial äußere Mantelfläche des Ausschütt- und Dosieranschlags 12 oder die genannte, radial äußere Spitze ist vorzugsweise zusätzlich mit einer auf der Oberfläche oder in einer Vertiefung an der Oberfläche angebrachten oder in dem Material gebildeten dünnen Markierungslinie versehen, die vorteilhafterweise fluoreszierend sein kann.

Wie in Verbindung mit den Figuren 9 bis 12 und insbesondere in Figur 13 erkennbar, weist der hintere Gehäuseabschnitt 3 über die Länge eines maximalen Ausschütthubs Ahmax eine Dosisskala 30 auf. Die Dosisskala 30 kann auf der Innenmantelfläche oder bevorzugter der Außenmantelfläche angebracht oder in dem durchsichtigen Mantel des hinteren Gehäuseabschnitts 3 eingelassen sein. Im Ausführungsbeispiel ist sie auf der äußeren Mantelfläche angebracht. Die Dosisskala 30 wird gebildet von Dosiszahlen, die Dosiseinheiten bezeichnen, und Dosismarken 31. Jeder der Dosiszahlen ist je eine der Dosismarken 31 zugeordnet. Die Dosismarken 31 werden von kurzen, dünnen Linien gebildet, die sich je in Umfangsrichtung des hinteren Gehäuseabschnitts 3 erstrecken. Die Dosiszahlen sind nach hinten aufsteigend in Form einer Spirale um die Rotationsachse L des Dosierglieds 13/14 über den Umfang des Gehäuseabschnitts 3 angeordnet. Entsprechend ergibt sich die spiralige Anordnung der Dosismarken 31 derart, dass jede der Dosismarken 31 eine andere axiale Höhe aufweist als jede der anderen Dosismarken 31. Die Dosismarken 31 bilden somit in feiner axialer Stufung jede in ganzen Dosiseinheiten auswählbare und ausschüttbare Produktdosis ab. Aufgrund der Durchsichtigkeit des hinteren Gehäuseabschnitts 3 kann die axiale Position der Antriebsvorrichtung 10/18 abgelesen werden, nämlich als die axiale Position, die der Zeiger 32 der Antriebsvorrichtung 10/18 relativ zu den Dosismarken 31 einnimmt. Im Ausführungsbeispiel bildet die genannte Markierungslinie, die an der radial äußeren Spitze des Ausschütt- und Dosieranschlags 12 um die Längsachse L umläuft, den Zeiger 32 der Antriebsvorrichtung 10/18.

Um die Ablesung der Dosisskala 30 zu erleichtern, insbesondere für Menschen mit geringer Sehschärfe, weist der äußere Dosierkörper 14 in einem Mantelabschnitt, der die Dosisskala 30 überdeckt, ein Sichtfenster 34 auf, das zu einer Lupe 34a weitergebildet ist. Das Sichtfenster 34 ist so groß und von solcher Form, dass genau eine der Dosiszahlen der Dosisskala 30 als die ausgewählte Dosis durch das Sichtfenster 34 identifiziert werden kann. Im Ausführungsbeispiel kann stets nur genau eine Dosiszahl durch den Sichtfenster abgelesen werden.

Damit die Dosisauswahl in diskreten Schritten, die den Dosiszahlen der Dosisskala 30 entsprechen, vorgenommen wird, sind das Dosierglied 13/14 und der hintere Gehäuseabschnitt 3 in diskreten Drehwinkelpositionen, die das Dosierglieds 13/14 relativ zu dem Gehäuseabschnitt 3 einnehmen kann, je in einem lösbaren Rasteingriff. Der Rasteingriff wird zwischen axial sich erstreckenden Nuten 7 und in die Nuten 7 eingreifenden Eingriffsgliedern 17 gebildet. Im Ausführungsbeispiel sind die axialen Nuten 7 an der äußeren Mantelfläche in den hinteren Gehäuseabschnitt 3 eingelassen. Entsprechend sind die Eingriffselemente 17 als kurze Rastnocken an der zugewandt inneren Mantelfläche des äußeren Dosierkörpers 14 geformt. Die Eingriffselemente 17 werden in den Axialnuten 7 in jeder der diskreten Drehwinkelpositionen des Dosierglieds 13/14 axial lineargeführt.

Eine hintere Endposition des Dosierglieds 13/14 wird durch Anschlag der Eingriffsglieder 17 an einer hinteren Stirnbegrenzungsfläche der Axialnuten 7 definiert. Eine vordere Endposition des Dosierglieds 13/14 wird durch Anschlag eines hinteren Verbindungsstegs, den die Dosierkörper 13 und 14 zwischen sich bilden, gegen eine hintere Stirnfläche des hinteren Gehäuseabschnitts 3 definiert. Zwischen diesen beiden extremen Dosierpositionen kann das Dosierglied 13/14 von Drehwinkelrastposition zu Drehwinkelrastposition hin und her verstellt werden, um den Dosieranschlag 15 axial zu verstellen. Der maximale Ausschütthub Ahmax der Antriebsvorrichtung 10/18 ist so groß wie der axiale Abstand, den der Dosieranschlag 15 in der hinteren Endstellung des Dosierglieds 13/14 von dem Ausschüttanschlag 5 aufweist abzüglich der axialen Stärke des Ausschütt- und Dosieranschlags 12.

Obgleich der Verwender die eingestellte Produktdosis jederzeit frei zwischen den beiden extremen Dosierpositionen verändern kann, ist für die meisten Anwendungen vorteilhaft, dass eine einmal eingestellte Dosis nicht mehr verändert werden muss. Das Injektionsgerät ist daher einerseits flexibel auf die Bedürfnisse unterschiedlicher Verwender einstellbar und andererseits in der für einen bestimmten Verwender optimalen Einstellung für eine wiederholte Verabreichung der jeweils gleichen Produktdosis verwendbar. In diesem Sinne wird durch das kombiniert rotatorisch und translatorisch relativ zu dem hinteren Gehäuseabschnitt 3 bewegbare und dadurch verstellbare Dosierglied 13/14 auch gleich ein Dosismemory erhalten.

Ein Vorteil, der durchaus nicht zu vernachlässigen ist, besteht darin, dass die Maßhaltigkeit und einfache Kontrolle der Maßhaltigkeit und die stabile Bauweise der Komponenten, die für die Dosierung und Ausschüttung maßgeblich sind, mit sehr wenigen Komponenten realisiert werden. Dies trägt dazu bei, dass das Gerät einfach aufgebaut und nicht zuletzt deshalb preiswert ist, aber dennoch präzise arbeitet und eine exakte Dosierung gewährleistet.

Vorteilhaft ist auch die ineinander geschachtelte Anordnung der Dosierkörper 13 und 14, wodurch eine Multifunktionalität des Dosierglieds 13/14 trotz einfacher Konstruktion erhalten wird. So bildet das Dosierglied 13/14 unmittelbar den Dosiereingriff mit dem Gehäuse des Injektionsgeräts, den Dosieranschlag 15 für die Antriebsvorrichtung 10/18, ein Griffteil für den Verwender und einen Teil einer doppelten Dosisanzeige, nämlich zum einen die Anzeige der ausgewählten Dosis und zum anderen die Anzeige der ausschüttbaren Dosis.

Als einen weiteren Vorteil ermöglicht das Injektionsgerät ein einfaches und sicheres Primen, d.h. ein Entlüften der produktführenden Teile zwischen dem Kolben K und der Austrittsöffnung der Injektionsnadel N.

Zur Erläuterung der Primingfunktion wird auf die Figuren 1 und 2 und insbesondere auch auf die Figuren 3, 4 und 5 hingewiesen. Um die Primingfunktion zu erfüllen, sind der innere Dosierkörper 13 und das Betätigungselement 18 in einem Eingriff. Der Eingriffbesteht zwischen einer Führungskurve und einem in die Führungskurve eingreifenden Eingriffsglied. Das Eingriffsglied ist in der Führungskurve zwischen Axialanschlägen einerseits und Radialanschlägen andererseits in einer definierten Art und Weise bewegbar. Die Führungskurve zum einen und das Eingriffsglied zum anderen sind an den einander zugewandten Mantelflächen des inneren Dosierkörpers 13 und des Betätigungselements 18 gebildet.

Wie in den Figuren 3 und 4 gezeigt, ist das mit 19 bezeichnete Eingriffsglied an einer Au-βenmantelfläche des Betätigungselements 18 geformt, während die mit 20 bezeichnete Führungskurve in die zugewandte Innenmantelfläche des inneren Dosierkörpers 13 eingelassen ist. Die Führungskurve 20 weist einen langen Axialabschnitt 21 und einen demgegenüber kürzeren Axialabschnitt 22 und einen Verbindungsabschnitt 24 auf, der sich in Umfangsrichtung erstreckt und die beiden Axialabschnitte 21 und 22 an ihren hinteren Enden miteinander verbindet. Die beiden im Bereich des Verbindungsabschnitts 24 in Umfangsrichtung sich gegenüberliegenden Seitenwände der Axialabschnitte 21 und 22 bilden Rotationsanschläge 25 und 26 für das Eingriffsglied 19. Der lange Axialabschnitt 21 erstreckt sich in Vorschubrichtung bis zu dem Dosieranschlag 15, genauer gesagt bis zu der hinteren Stirnfläche der Ringschulter, die den Dosieranschlag 15 bildet. Der kürzere Axialabschnitt 22 verläuft parallel zu dem Axialabschnitt 21 und ist als kurze Sacknut ausgebildet. Eine vordere Stirnfläche des Axialabschnitts 22 bildet einen Priminganschlag 23. Die Axialabschnitte 21 und 22 münden an dem hinteren Stirnende des inneren Dosierkörpers 13. Der Verbindungsabschnitt 24 ist entsprechend an dem hinteren Stirnende offen. Das Eingriffsglied 19 wird von einer Axialrippe gebildet, die an dem Außenmantel des Betätigungselements 18 geformt ist. Diese Axialrippe bildet den Priminggegenanschlag an ihrer freien vorderen Stirnfläche.

Durch den Eingriff des Eingriffsglieds 19 in die derart gebildete Führungskurve 20 kann das Betätigungselement 18 relativ zu dem inneren Dosierkörper 13 die der Führungskurve 20 entsprechende Form der Bewegung ausführen, nämlich jeden ausgewählten Ausschütthub Ah bis hin zum maximalen Ausschütthub Ahmax, den demgegenüber kürzeren Priminghub Ph und die rotatorische Auswahlbewegung. Da das Betätigungselement 18 mit dem Antriebsglied 10 axial nicht bewegbar, aber um die Längsachse L verdrehbar verbunden ist, kann das Betätigungselement 18 ohne Einwirkung auf das Antriebsglied 10 zwischen den beiden Rotationsanschlägen 25 und 26 hin und her gedreht werden. Die axiale Translationsbewegung in entweder dem langen Axialabschnitt 21 oder dem kurzen Axialabschnitt 22 ist dann jedoch nur zusammen mit dem Antriebsglied 10 möglich.

Die Rotationsbewegung des Betätigungselements 18 zwischen den beiden Rotationsanschlägen 25 und 26, d.h. aus der Ausschüttposition in die Primingposition und umgekehrt, ist wegen der Anordnung des Verbindungsabschnitts 24 an den hinteren Enden der Axialabschnitte 21 und 22 nur möglich, wenn das Antriebsglied 10 mit seinem Ausschütt- und Dosieranschlag 12 auf Anschlag gegen den Dosieranschlag 15 liegt. In dieser "geladenen" Stellung kann das Eingriffsglied 19 durch Verdrehung des Betätigungselements 18 relativ zu dem inneren Dosierkörper 13 gegen den Rotationsanschlag 25 und somit in die axiale Flucht zu dem Priminganschlag 23 bewegt werden. In dieser Drehwinkelposition des Betätigungselements 18, der Primingposition, kann die Antriebsvorrichtung 10/18 durch axialen Druck auf das Betätigungselement 18 bis gegen den Priminganschlag 23 in Vorschubrichtung bewegt werden. Die axiale Länge Ph dieses Priminghubs beträgt nur wenige Dosiseinheiten, beispielsweise zwei, drei oder vier Dosiseinheiten. Entsprechend kurz ist der axiale Abstand zwischen dem Priminganschlag 23 und dem Priminggegenanschlag, den das Eingriffsglied 19 bildet.

Vorteilhaft ist es, wenn das Betätigungselement 18 mit dem inneren Dosierkörper 13 in der Flucht der Axialabschnitte 21 und 22, d.h. an den Rotationsanschlägen 25 und 26, jeweils in einem lösbaren Rasteingriff ist. Um in der Ausschüttposition und in der Primingposition je den Rasteingriff mit dem inneren Dosierkörper 13 zu erhalten, ist das Eingriffsglied 19 an seiner schmalen äußeren Mantelfläche nochmals mit einem dünnen, axialen Rastnocken versehen, der in den beiden Rotationsanschlagpositionen des Betätigungselements 18 jeweils in einer von zwei entsprechend in der Führungskurve 20 geformten, axialen Rastnuten zu liegen kommt.

Im Ausführungsbeispiel sind zwei identische Führungskurven 20 und Eingriffsglieder 19 vorgesehen, die einander diametral gegenüberliegen.

In der Variante der Figur 4 verläuft der Verbindungsabschnitt 24 auf einer einzigen axialen Höhe einfach gerade.

Figur 5 zeigt eine Führungskurve 20 mit einem Verbindungsabschnitt 24', dessen vordere Führungswand von dem Priminganschlag 23 aus schräg nach hinten in den langen Axialabschnitt 21 führt. Aufgrund des schrägen Verlaufs des Verbindungsabschnitts 24' wird bei der Rückdrehbewegung des Betätigungselements 18 von dem Rotationsanschlag 25 auf den Rotationsanschlag 26 zu eine Translationsbewegung des Betätigungselements 18 und damit zusammen des Antriebsglieds 10 gegen die Vorschubrichtung und relativ zu der Kolbenstange 8 bewirkt. Nach der Ausführung eines Primingvorgangs muss der Verwender deshalb die Antriebsvorrichtung 10/18 nicht wieder extra relativ zu der Kolbenstange 8 zurückziehen, d.h. laden. Die Aufzieh- bzw. Ladebewegung wird vielmehr durch die Rückdrehbewegung erzwungen. Ansonsten entspricht die Führungskurve 20 der Figur 5 der Führungskurve 20 der Figur 4.

Nachfolgend wird die Funktionsweise des Injektionsgeräts beschrieben.

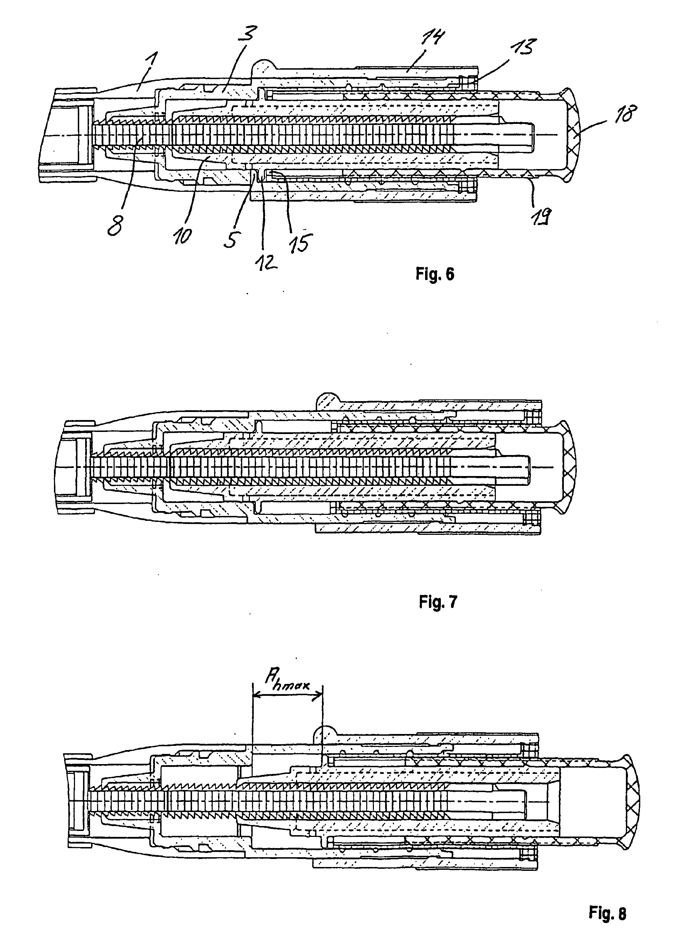

Die Figuren 6 und 9 zeigen den hinteren Teil des Injektionsgeräts in einem Längsschnitt und in einer Ansicht je in einem Zustand, aus dem heraus die Dosisauswahl vorgenommen wird. Dementsprechend liegt die Antriebsvorrichtung 10/18 auf Anschlag gegen den Ausschüttanschlag 5, und das Dosierglied 13/14 nimmt seine axial vordere Endposition ein. Zwischen dem Ausschütt- und Dosieranschlag 12 der Antriebsvorrichtung 10/18 und dem Dosieranschlag 15 des Dosierglieds 13/14 verbleibt ein lichter Abstand, der zwei Dosiseinheiten entspricht. Demgemäß kann in dem Sichtfenster 34 die Dosis von zwei Dosiseinheiten abgelesen werden. Deutlich erkennbar ist ferner auch die dieser Dosis zugeordnete Dosismarke 31. Da die Antriebsvorrichtung 10/18 mit ihrem Ausschütt- und Dosieranschlag 12 gegen den Ausschüttanschlag 5 auf Anschlag liegt, verläuft die den Zeiger 32 bildende Markierungslinie der Antriebsvorrichtung 10/18 parallel mit einem axialen Abstand zu der Dosismarke 31. Der Abstand entspricht zwei Dosiseinheiten. Diese Situation ist in Figur 9 erkennbar. Durch Zurückziehen der Antriebsvorrichtung 10/18 relativ zu der Kolbenstange 8 bis gegen den Dosieranschlag 15 wird das Injektionsgerät "geladen". Wenn diese Ladebewegung durchgeführt ist, können zwei Dosiseinheiten durch anschließendes Vorschieben der Antriebsvorrichtung 10/18 und der dabei mitgenommenen Kolbenstange 8 ausgeschüttet werden. Figur 10 zeigt diesen Zustand, in dem die Antriebsvorrichtung 10/18 mit ihrem Ausschütt- und Dosieranschlag 12 gegen den Dosieranschlag 15 des Dosierglieds 13/14 auf Anschlag liegt. Die der Dosiszahl "2" zugeordnete Dosismarke 31 und der Zeiger 32 überdecken einander exakt.

Aus dem in den Figuren 6 und 9 dargestellten Zustand heraus nimmt der Verwender seine Dosisauswahl vor. Für die Dosisauswahl muss lediglich das Dosierglied 13/14 um die Längsachse L relativ zu dem hinteren Gehäuseabschnitt 3 verdreht werden. Hierbei bilden der äußere Dosierkörper 14 ein Griffteil und der innere Dosiskörper 13 eine Dosierschraube, deren Drehbewegung durch den Gewindeeingriff unmittelbar in die axiale Verstellbewegung zum Zwecke der Dosisauswahl umgewandelt wird. Dabei erfolgt die Dosierung in diskreten Drehwinkelrastpositionen, die das Dosierglied 13/14 relativ zu dem hinteren Gehäuseabschnitt 3 sukzessive einnimmt.

Die Figuren 7 und 11 zeigen das Injektionsgerät unmittelbar nach der Dosisauswahl. Eingestellt ist eine Dosis von 42 Dosiseinheiten, wie im Sichtfenster 34 in Figur 11 abgelesen werden kann. In dem in Figur 7 dargestellten Zustand ist die Dosisauswahl abgeschlossen, das Injektionsgerät ist jedoch noch nicht "geladen". Die Antriebsvorrichtung 10/18 liegt nämlich noch auf Anschlag gegen den Ausschüttanschlag 5. Dies wird durch den Zeiger 32 auf der Dosisskala 30 angezeigt, wie in Figur 11 zu erkennen ist.

Die Figur 8 zeigt eine Maximaldosierung. Fig. 12 zeigt das Injektionsgerät in einem Zustand, in dem die Dosisauswahl abgeschlossen ist, aber die Antriebsvorrichtung 10/18 nicht um den hierdurch definierten Ausschütthub der Länge Ah, sondern nur um einen Teil dieses Hubs zurückgezogen wurde. In dem in Fig. 12 beispielhaft dargestellten Zustand wurde die Antriebsvorrichtung 10/18 um einen Hub zurückgezogen, der einer Produktdosis von acht Dosiseinheiten entspricht. Dies zeigt die axiale Position des Zeiger 32 auf der Dosisskala 30 in Figur 12 an.

Wie Figur 12 verdeutlicht, wird durch die Durchsichtigkeit des hinteren Gehäuseabschnitts 3, die Bildung des Zeigers 32 und die Anordnung der Dosisskala 30 eine einfache, aber zuverlässige Restmengenanzeige erhalten. Enthält die Ampulle 2 nämlich vor dem Zurückziehen der Antriebsvorrichtung 10/18 nicht mehr die volle ausgewählte Produktdosis von beispielsweise 42 Dosiseinheiten, so kann die Antriebsvorrichtung 10/18 gegen die Vorschubrichtung nur soweit zurückgezogen werden, dass die Länge ihres nächsten Ausschütthubs in die Vorschubrichtung der Produktdosis entspricht, die in der Ampulle 2 für eine letzte Ausschüttung noch verfügbar ist. Im Ausführungsbeispiel beträgt diese Restmenge acht Dosiseinheiten.

Dementsprechend überdecken sich der Zeiger 32 und die Dosismarke 31, die der Dosis von acht Dosiseinheiten zugeordnet ist. Die ausschüttbare Restmenge wird durch einen Anschlag definiert, der zwischen der Kolbenstange 8 und der Antriebsvorrichtung 10/18 wirksam ist. Dieser Anschlag begrenzt die effektive Länge der Kolbenstange 8, d.h. die Länge, um die die Kolbenstange 8 aus ihrer Position vor einer ersten Ausschüttung insgesamt bis zur Entleerung der Ampulle 2 in die Vorschubrichtung bewegt werden kann. Im Ausführungsbeispiel bildet die Kolbenstange 8 in ihrem hinteren Teil diesen Anschlag für die Mitnehmer 11, d.h. die Mitnehmer 11 können diesem Anschlag nicht elastisch ausweichen.

### Bezugszeichenliste:

- 1: erster oder vorderer Gehäuseabschnitt
- 2: Reservoir, Ampulle
- 3: zweiter oder hinterer Gehäuseabschnitt
- 4: Sperreinrichtung
- 5: Ausschüttanschlag
- 6: Gewinde
- 7: erstes Rastelement, Axialnut
- 8: Kolbenstange
- 10: Antriebsglied
- 11: Mitnehmer
- 12: Ausschütt- und Dosieranschlag
- 13: Dosierglied, innerer Dosierkörper
- 14: Dosierglied, äußerer Dosierkörper
- 15: Dosieranschlag
- 16: Gewinde
- 17: zweites Rastelement, Eingriffsglied
- 18: Betätigungselement
- 19: Priminggegenanschlag, Eingriffsglied
- 20: Führungskurve
- 21: erster Führungsabschnitt
- 22: zweiter Führungsabschnitt
- 23: Priminganschlag
- 24: Verbindungsabschnitt
- 25: Rotationsanschlag
- 26: Rotationsanschlag
- 30: Dosisskala
- 31: Dosismarke
- 32: Zeiger, Markierungslinie
- 33: -
- 34: Sichtfenster
- 34a: Lupe
- K: Kolben
- L: Translationsachse, Rotationsachse, Längsachse
- N: Injektionsnadel
- P: Schutzkappe

## Patentansprüche

1. Verabreichungsgerät für die Verabreichung einer auswählbaren Dosis eines fluiden Produkts, das Verabreichungsgerät umfassend:
a) ein Gehäuse (1, 3) mit einem ersten Gehäuseabschnitt (1), der ein Reservoir (2) für das Produkt aufnimmt, und einem zweiten Gehäuseabschnitt (3),
b) eine Abtriebsvorrichtung (K, 8), die auf das in dem Reservoir (2) enthaltene Produkt wirkt, um das Produkt auszuschütten,
c) ein Dosierglied (13/14), das einen Dosieranschlag (15) bildet und für eine Verstellung des Dosieranschlags (15), durch die die Produktdosis ausgewählt wird, bewegbar mit dem Gehäuse (1, 3) gekoppelt ist,
d) und eine auf die Abtriebsvorrichtung (K, 8) wirkende Antriebsvorrichtung (10/18), die von dem zweiten Gehäuseabschnitt (3) aufgenommen wird und in eine Antriebsrichtung bis gegen einen Ausschüttanschlag (5) einen die Abtriebsvorrichtung (K, 8) antreibenden Ausschütthub (Ah) ausführt und gegen die Antriebsrichtung bis gegen den Dosieranschlag (15) bewegbar ist,
e) wobei das Dosierglied (13/14) für die Verstellung des Dosieranschlags (15) in und/oder gegen die Antriebsrichtung translatorisch bewegbar ist,
f) und wobei das Dosierglied (13/14) und der zweite Gehäuseabschnitt (3) über ein Gelenk (6, 16) so gekoppelt sind, dass eine Rotationsbewegung des Dosierglieds (13/14), die das Dosierglied relativ zu dem Gehäuse (1, 3) ausführt, die translatorische Bewegung des Dosierglieds (13/14) bewirkt.

2. Verabreichungsgerät nach Anspruch 1, **dadurch gekennzeichnet, dass** das Verabreichungsgerät eine Dosisskala (30) aufweist und das Dosierglied (13/14) ein Sichtfenster (34) bildet, durch das die ausgewählte Produktdosis auf der Dosiskala (30) abgelesen werden kann.

3. Verabreichungsgerät nach einem der vorhergehenden Ansprüche, **dadurch gekennzeichnet, dass** der Dosieranschlag (15) an einem bezogen auf die Antriebsrichtung vorderen Ende des Dosierglieds (13/14) gebildet ist, vorzugsweise einen vorderen Hülsenrand des Dosierglieds (13/14) bildet.

4. Verabreichungsgerät nach einem der vorhergehenden Ansprüche, **dadurch gekennzeichnet, dass** die Antriebsvorrichtung (10/18) wenigstens einen Mitnehmer (11) bildet, der mit der Abtriebsvorrichtung (K, 8) in einem Eingriff steht, der eine Mitnahme der Abtriebsvorrichtung (K, 8) durch die Antriebsvorrichtung (10/18) in die Antriebsrichtung bewirkt, wobei der Eingriff gegen eine elastische Rückstellkraft gelöst wird, um die Antriebsvorrichtung (10/18) relativ zu der Abtriebsvorrichtung (K, 8) gegen die Antriebsrichtung zu bewegen.

5. Verabreichungsgerät nach dem vorhergehenden Anspruch, **dadurch gekennzeichnet, dass** der wenigstens eine Mitnehmer (11) in dem Eingriff frei von dem Dosierglied (13/14) ist.

6. Verabreichungsgerät nach einem der zwei vorhergehenden Ansprüche, **dadurch gekennzeichnet, dass** der wenigstens eine Mitnehmer (11) in dem Eingriff mit der Abtriebsvorrichtung (K, 8) ist, wenn die Antriebsvorrichtung (10/18) auf Anschlag gegen den Dosieranschlag (15) ist.

7. Verabreichungsgerät nach einem der drei vorhergehenden Ansprüche, **dadurch gekennzeichnet, dass** die Antriebsvorrichtung (10/18) für den Dosieranschlag (15) einen starren Dosiergegenanschlag (12) bildet, relativ zu dem der wenigstens eine Mitnehmer (11) gegen die elastische Rückstellkraft bewegbar ist.

8. Verabreichungsgerät nach einem der vier vorhergehenden Ansprüche, **dadurch gekennzeichnet, dass** der wenigstens eine Mitnehmer (11) in einem Abschnitt der Antriebsvorrichtung (10/18) gebildet ist, der in Antriebsrichtung über das Dosierglied (13/14) hinausragt.

9. Verabreichungsgerät nach einem der fünf vorhergehenden Ansprüche, **dadurch gekennzeichnet, dass** das Verabreichungsgerät eine Dosisskala (30) aufweist und dass die Antriebsvorrichtung (10/18) einen Zeiger für die Dosisskala (30) bildet oder mit der Dosisskala versehen ist.

10. Verabreichungsgerät nach einem der vorhergehenden Ansprüche, **dadurch gekennzeichnet, dass** die Antriebsvorrichtung (10/18) das hohlzylindrische Dosierglied (13/14) durchragt.

11. Verabreichungsgerät nach einem der vorhergehenden Ansprüche, **dadurch gekennzeichnet, dass** das Dosierglied (13/14) einen inneren Dosierkörper (13) und einen äußeren Dosierkörper (14) umfasst und der innere Dosierkörper (13) den Dosieranschlag (15) und der äußere Dosierkörper (14) ein Griffteil für eine die Verstellung des Dosieranschlags (15) bewirkende Betätigung des Dosierglieds (13/14) bildet.

12. Verabreichungsgerät nach dem vorhergehenden Anspruch, **dadurch gekennzeichnet, dass** zwischen dem inneren Dosierkörper (13) und dem äußeren Dosierkörper (14) ein Spalt gebildet ist, in den das Gehäuse (1, 3) ragt.

13. Verabreichungsgerät nach einem der vorhergehenden Ansprüche, **dadurch gekennzeichnet, dass** das Dosierglied (13/14) und das Gehäuse (1, 3) in diskreten Dosierpositionen des Dosierglieds (13/14) je in einem lösbaren Eingriff, vorzugsweise Rasteingriff, sind.

14. Verabreichungsgerät nach einem der vorhergehenden Ansprüche, **dadurch gekennzeichnet, dass** das eine aus Dosierglied (13/14) und Gehäuse (1, 3) mehrere in Antriebsrichtung sich erstreckende Rippen oder Vertiefungen (7) bildet und das andere aus Dosierglied (13/14) und Gehäuse (1, 3) wenigstens ein Eingriffselement (17) bildet, das in den diskreten Dosierpositionen des Dosierglieds (13/14) mit wenigstens einer der Vertiefungen (7) oder Rippen in dem lösbaren Eingriff ist.

15. Verabreichungsgerät nach dem vorhergehenden Anspruch, **dadurch gekennzeichnet, dass** die Vertiefungen (7) oder Rippen je einen Translationsanschlag bilden, gegen den das wenigstens eine Eingriffselement (17) bewegbar ist, um eine auswählbare Maximaldosis zu bestimmen.

16. Verabreichungsgerät nach einem der vorhergehenden Ansprüche, **dadurch gekennzeichnet, dass** ein in dem Reservoir (2) in die Antriebsrichtung bewegbar aufgenommener Kolben (K) und eine in die Antriebsrichtung auf den Kolben (K) wirkende Zahnstange die Abtriebsvorrichtung (K, 8) bilden oder mitbilden.

17. Verabreichungsgerät nach einem der vorhergehenden Ansprüche, **dadurch gekennzeichnet, dass** es eine Injektionsnadel (N) von 30 G oder 31 G aufweist.

## Claims

1. An administration device for the administration of a selectable dose of a fluid product, the administration device including:
a) a housing (1, 3) with a first housing portion (1) which accommodates a reservoir (2) for the product, and a second housing portion (3),
b) a driven device (K, 8) which acts on the product contained in the reservoir (2) to expel the product,
c) a metering member (13/14) which forms a metering abutment (15) and which is coupled movably to the housing (1, 3) for a displacement of the metering abutment (15) by which the product dose is selected, and
d) a drive device (10/18) which acts on the driven device (K, 8) and which is accommodated by the second housing portion (3) and which performs an expulsion stroke movement (Ah) for driving the driven device (K, 8) in a drive direction until encountering an expulsion abutment (5) and is movable in opposite relationship to the drive direction until encountering the metering abutment (15),
e) wherein the metering member (13/14) for displacement of the metering abutment (15) is movable with a translatory movement in and/or in opposite relationship to the drive direction,
f) and wherein the metering member (13/14) and the second housing portion (3) are coupled by way of a pivot (6, 16) in such a way that a rotary movement of the metering member (13/14) which the metering member performs relative to the housing (1, 3) produces the translatory movement of the metering member (13/14).

2. An administration device according to claim 1 **characterised in that** the administration device has a dose scale (30) and the metering member (13/14) forms a viewing window (34) through which the selected product dose can be read off on the dose scale (30).

3. An administration device according to one of the preceding claims **characterised in that** the metering abutment (15) is formed at an end of the metering member (13/14), which is the front end with respect to the drive direction, and preferably forms a front sleeve edge of the metering member (13/14).

4. An administration device according to one of the preceding claims **characterised in that** the drive device (10/18) forms at least one entrainment portion (11) which is in engagement with the driven device (K, 8), which causes entrainment of the driven device (K, 8) by the drive device (10/18) in the drive direction, wherein the engagement is released against a resilient return force in order to move the drive device (10/18) relative to the driven device (k, 8) in opposite relationship to the drive direction.

5. An administration device according to the preceding claim **characterised in that** the at least one entrainment portion (11) in the engagement is free from the metering member (13/14).

6. An administration device according to one of the two preceding claims **characterised in that** the at least one entrainment portion (11) is in engagement with the driven device (K, 8) when the drive device (10/18) is in a position of abutment against the metering abutment (15).

7. An administration device according to one of the three preceding claims **characterised in that** the drive device (10/18) for the metering abutment (15) forms a rigid metering counter-abutment (12), relative to which the at least one entrainment portion (11) is movable against the elastic return force.

8. An administration device according to one of the four preceding claims **characterised in that** the at least one entrainment portion (11) is formed in a portion of the drive device (10/18) which projects in the drive direction beyond the metering member (13/14).

9. An administration device according to one of the five preceding claims **characterised in that** the administration device has a dose scale (30) and that the drive device (10/18) forms a pointer for the dose scale (30) or is provided with the dose scale.

10. An administration device according to one of the preceding claims **characterised in that** the drive device (10/18) projects through the hollow-cylindrical metering member (13/14).

11. An administration device according to one of the preceding claims **characterised in that** the metering member (13/14) includes an inner metering body (13) and an outer metering body (14) and the inner metering body (13) forms the metering abutment (15) and the outer metering body (14) forms a handle for actuation of the metering member (13/14), for causing the displacement of the metering abutment (15).

12. An administration device according to the preceding claims **characterised in that** formed between the inner metering body (13) and the outer metering body (14) is a gap into which the housing (1, 3) projects.

13. An administration device according to one of the preceding claims **characterised in that** the metering member (13/14) and the housing (1, 3) in discrete metering positions of the metering member (13/14) are respectively in releasable engagement, preferably latching engagement.

14. An administration device according to one of the preceding claims **characterised in that** one of the metering member (13/14) and the housing (1/3) forms a plurality of ribs or depressions (7) extending in the drive direction and the other of the metering member (13/14) and the housing (1, 3) forms at least one engagement element (17) which in the discrete metering positions of the metering member (13/14) is in releasable engagement with at least one of the depressions (7) or ribs.

15. An administration device according to the preceding claim **characterised in that** the depressions (7) or ribs form a respective translation abutment against which the at least one engagement element (17) is movable to determine a selectable maximum dose.

16. An administration device according to one of the preceding claims **characterised in that** a piston (K) accommodated in the reservoir (2) movably in the drive direction and a toothed rack acting on the piston (K) in the drive direction form or jointly form the driven device (K, 8).

17. An administration device according to one of the preceding claims **characterised in that** it has an injection needle (N) of 30 G or 31 G.

## Revendications

1. Appareil d'administration pour l'administration d'une dose pouvant être choisie d'un produit liquide, l'appareil d'administration comprenant :
a) un boîtier (1, 3) avec une première section de boîtier (1) qui loge un réservoir (2) pour le produit et une deuxième section de boîtier (3),
b) un dispositif de sortie (K, 8) qui agit sur le produit contenu dans le réservoir (2) pour déverser le produit,
c) un élément de dosage (13/14) qui forme une butée de dosage (15) et est couplé de manière mobile avec le bâti (1, 3) pour régler la butée de dosage (15) par le biais de laquelle la dose de produit est sélectionnée,
d) et un dispositif d'entraînement (10/18) qui agit sur le dispositif de sortie (K, 8), et qui est logé dans la deuxième section de boîtier (3) et effectue dans un sens d'entraînement jusque contre une butée de déversement (5) une course de déversement (Ah) qui entraîne le dispositif de sortie (K, 8) et peut être déplacé dans le sens contraire à celui de l'entraînement jusque contre la butée de dosage (15),
e) l'élément de dosage (13/14) pour le réglage de la butée de dosage (15) pouvant être déplacé par translation dans le sens de l'entraînement et/ou dans le sens contraire, f) et l'élément de dosage (13/14) et la deuxième section de boîtier (3) étant couplés via une articulation (6, 16) de telle manière qu'un mouvement de rotation de l'élément de dosage (13/14) que l'élément de dosage effectue par rapport au boîtier (1, 3) provoque le mouvement de translation de l'élément de dosage (13/14).

2. Appareil d'administration selon la revendication 1, **caractérisé en ce que** l'appareil d'administration comporte une graduation de dosage (30) et l'élément de dosage (13/14) forme une fenêtre d'inspection (34) à travers laquelle la dose de produit sélectionnée peut être lue sur la graduation de dosage (30).

3. Appareil d'administration selon une des revendications précédentes, **caractérisé en ce que** la butée de dosage (15) est formée à une extrémité avant de l'élément de dosage (13/14) vu dans le sens de l'entraînement et forme de préférence un bord à collet avant de l'élément de dosage (13/14).

4. Appareil d'administration selon une des revendications précédentes, **caractérisé en ce que** le dispositif d'entraînement (10/18) forme au moins un entraîneur (11) qui est en prise avec le dispositif de sortie (K, 8), la prise provoquant un entraînement du dispositif de sortie (K, 8) par le dispositif d'entraînement (10/18) dans le sens d'entraînement, la prise étant détachée contre une force de rappel afin de déplacer le dispositif d'entraînement (10/18) par rapport au dispositif de sortie (K, 8) dans le sens contraire au sens d'entraînement.

5. Appareil d'administration selon la revendication précédente, **caractérisé en ce que** le au moins un entraîneur (11) est exempt de l'élément de dosage (13/14) en prise.

6. Appareil d'administration selon une des deux revendications précédentes, **caractérisé en ce que** le au moins un entraîneur (11) est en prise avec le dispositif de sortie (K, 8) lorsque le dispositif d'entraînement (10/18) est en butée contre la butée de dosage (15).

7. Appareil d'administration selon une des trois revendications précédentes, **caractérisé en ce que** le dispositif d'entraînement (10/18) forme pour la butée de dosage (15) une contre-butée de dosage rigide (12) par rapport à laquelle le au moins un entraîneur (11) peut être déplacé contre la force de rappel élastique.

8. Appareil d'administration selon une des quatre revendications précédentes, **caractérisé en ce que** le au moins un entraîneur (11) est formé dans une section du dispositif d'entraînement (10/18) qui dépasse de l'élément de dosage (13/14) dans le sens d'entraînement.

9. Appareil d'administration selon une des cinq revendications précédentes, **caractérisé en ce que** l'appareil d'administration comporte une graduation de dosage (30) et **en ce que** le dispositif d'entraînement (10/18) forme une aiguille pour la graduation de dosage (30) ou est doté de la graduation de dosage.

10. Appareil d'administration selon une des revendications précédentes, **caractérisé en ce que** le dispositif d'entraînement (10/18) traverse l'élément de dosage cylindrique (13/14).

11. Appareil d'administration selon une des revendications précédentes, **caractérisé en ce que** l'élément de dosage (13/14) comprend un corps de dosage intérieur (13) et un corps de dosage extérieur (14) et le corps de dosage intérieur (13) forme la butée de dosage (15) et le corps de dosage extérieur (14) un élément de poignée pour un actionnement de l'élément de dosage (13/14) qui permet de modifier le réglage de la butée de dosage (15).

12. Appareil d'administration selon la revendication précédente, **caractérisé en ce qu'**une fente dans laquelle le boîtier (1,3) pénètre est formée entre le corps de dosage intérieur (13) et le corps de dosage extérieur (14).

13. Appareil d'administration selon une des revendications précédentes, **caractérisé en ce que** l'élément de dosage (13/14) et le boîtier (1, 3) sont dans des positions de dosage discrètes de l'élément de dosage (13/14) chacun dans une prise amovible, de préférence une prise avec enclenchement.

14. Appareil d'administration selon une des revendications précédentes, **caractérisé en ce que** l'un de l'élément de dosage (13/14) et du boîtier (1, 3) forme plusieurs nervures ou creux (7) qui s'étendent dans le sens d'entraînement et **en ce que** l'autre de l'élément de dosage (13/14) et du boîtier (1, 3) forme au moins un élément de prise (17) qui, dans les positions de dosage discrètes de l'élément de dosage (13/14), est en prise amovible avec au moins un des creux (7) ou nervures.

15. Appareil d'administration selon la revendication précédente, **caractérisé en ce que** chaque creux (7) ou nervure forme une butée de translation contre laquelle le au moins un élément de prise (17) peut être déplacé afin de déterminer une dose maximale pouvant être sélectionnée.

16. Appareil d'administration selon une des revendications précédentes, **caractérisé en ce qu'**un piston (K) logé de façon déplaçable dans le sens de l'entraînement dans le réservoir (2) et une crémaillère agissant sur le piston (K) dans le sens d'entraînement forment ou contribuent à former le dispositif de sortie (K, 8).

17. Appareil d'administration selon une des revendications précédentes, **caractérisé en ce qu'**il comporte une aiguille d'injection (N) de 30 G ou 31 G.
